# EUROPEAN PATENT SPECIFICATION

(11) **EP 0 576 155 B1**
(45) Date of publication and mention of the grant of the patent: **09.09.1998**
(21) Application number: 93304128.7
(22) Date of filing: 27.05.1993
(51) Int. Cl.: G01N 33/60, B01D 25/00

(54) **Method and a filter for filtration of radioassays**
Verfahren und Filter für Filterung von Radioassays
Méthode et filtre pour des essais radiofiltrants

(30) Priority: 27.05.1992 US 888752
(43) Date of publication of application: 29.12.1993
(73) Proprietor: Potter, Colin Gerald, Headington Oxford OX3 7RR (GB); Warner, Gerald Truscott, Headington Oxford (GB)
(72) Inventor: Potter, Colin Gerald, Headington Oxford OX3 7RR (GB); Warner, Gerald Truscott, Headington Oxford (GB)
(74) Representative: Gallafent, Richard John

(56) References cited:
- WO-A-89/11664
- WO-A-90/11524
- DATABASE WPI Week 9144, Derwent Publications Ltd., London, GB; AN 91-321614 & JP-A-3 215 473 (SUMITOMO SEIYAKU KK) 20 September 1991

## Description

This invention relates to a method in filtration radio isotope competition assays and a filter in said assays.

Biological cells or membranes which have incorporated compounds labelled with radioactive isotopes are commonly separated from the unincorporated compound by means of filtration assays. The compounds in this application are organic, inorganic or biological compounds, elements or ions.

In one assay, cells previously incubated with tritiated thymidine, which is incorporated into synthesizing DNA, are filtered on to glass fibre filters. Filtration can also be used in ligand binding assays where there is a competitive assay of an unknown amount of ligand and a known amount of labelled ligand for receptors present on a membrane or cell preparation. Macromolecules such as nucleic acids can also be filtered on to fine porosity transfer membranes.

In any filtration assay, there needs to be an effective filtration of the particles which implies careful choice of filter porosity and composition - paper, glass fibre or nylon filters are commonly used. In addition, minimal non-specific absorption of non-incorporated compound is essential and this is governed by the surface properties of the filter material and the chemical composition of the assay solutions. Glass fibre has rather a reactive surface and sometimes the non-specific binding can be reduced by pre-treatment of the filter, for example by soaking in a solution of polyethyleneimine before use. High non-specific binding leads to a poor range of activity present on the filter and limits sensitivity of the assay. According to the invention, it is now described how more than one layer of filter materials may be used to improve the sensitivity of such assays.

The method in the present invention is characterised by using at least two filters for the same sample so that the radioactive count rate of the first filter will indicate the activity of the particles and that of a subsequent filter will estimate non-specific binding.

The filter in the present invention is characterised in that the filter comprises at least two filters which are used for the same sample so that the radioactive count rate of the first filter will indicate the activity of the particles and that of a subsequent filter will estimate non-specific binding.

If two identical filters are used one on top of the other in a filtration assay, the top filter may be such as to remove virtually all the particles although it inevitably also exhibits some non-specific binding properties. The second filter will just retain non-specific binding so that after washing the filters they may be separated, dried and counted using a suitable counter. It is thus possible to obtain count rates proportional to filtered (bound) + non-specific (free) radioactivity in the first filter and non-specific (free) in the second. For assays where the radioactive compound is of similar concentration in the particulate fraction as in solution, then at low concentrations of competing unlabelled compound, there may not be enough free radioactive material to give as high a count as possible since all the sites may not become filled and/or the reaction will slow as the solution is depleted. In the present technique, the ratio of activities on the two filters will therefore give a better calibration curve with greater dynamic range than the activities of the particles alone. Furthermore, any small errors in the amount of label or total assay placed in the reaction vessel will be less important if the ratio is measured rather than the absolute count rate of the particles.

In order to improve further this technique, it would be better to trap as much as possible on the second filter. This could be done by having a greater thickness to absorb more activity. If the compound is charged then a second filter with opposite charge will bind the material strongly. Carboxymethyl cellulose and DEAE coated filters would be useful here. Peptides could be trapped on cellulose phosphate coated filters and DNA on DEAE or charged nylon. Whatever method is used, this will also have the advantage that most of the material will be disposed of in the filters rather than being discharged with the washate to the drains.

The first filter could also be improved to reduce non-specific binding. Considering again if the free compound were charged, then a filter having the same charge will repel the molecules as they pass through the pores of the filter and make trapping less likely. DEAE, cellulose phosphate and carboxymethyl coated filters are typical and could be used. Hydrophobic filters may also reduce trapping of hydrophilic compounds.

The above methods require that the two filters are to be separated for counting. If the two filters are made with a coating of scintillant before surface treatment suitable for the assay and that the scintillant composition is different such that the light pulses emitted are different with regard to pulse height or length when measured in a liquid scintillation counter, then it would be possible to measure the count rate associated with the particles and the second filter separately by analysis of the different pulse lengths and/or heights, analogous to double isotope labelling. As an example, scintillant containing anthracene will have a higher energy tritium pulse height spectrum than one containing PPO. An analogous method has been used in double proximity assays but where there is no separation of bound and free, except by the proximity effect presented in the publication WO90/11524 corresponding to Finnish Patent 82144 by Potter, Warner & Oikari.

In the above scheme, the scintillant sheets need not necessarily be dried, but if they were and the scintillant was meltable as described in US Patent 4,972,084 by Potter & Warner, then this would permit increased counting efficiency for the radioactivity on both filters.

Of course, several sheets or layers could be used in series to filter different components in ways similar to that described above. Upper filters could be graded in porosity to separate different components according to size and lower filters could have different coatings suitable for the different compounds in solution.

## Claims

1. A method in filtration radio isotope competition assays, characterised by using at least two filters for the same sample so that the radioactive count rate of the first filter will indicate the activity of the particles and that of a subsequent filter will estimate non-specific binding.

2. A method according to Claim 1, characterised by using the ratio of the radioactivity of the filters as an estimate of bound versus free material to be compared with standard samples.

3. A Method according to Claim 1 or 2, characterised in that the filters are coated with or composed of scintillants with different composition so that labelled compounds incorporated into particles borne on a first filter will give different light characteristics from those of the free compound bound by a subsequent filter.

4. A method according to any one of Claims 1 to 3, characterised by drying and heating the filters after filtration but before counting to increase counting efficiency.

5. A filter for filtration in radio isotope competition assays characterised in that the filter comprises at least two filters which are used for the same sample so that the radioactive count rate of the first filter will indicate the activity of the particles and that of a subsequent filter will estimate non-specific binding.

6. A filter according to Claim 5, characterised in that the second filter is thicker than the first to absorb more free compound and increase counting accuracy where the compounds are organic, inorganic or biological compounds, elements or ions.

7. A filter according to Claim 5 or 6, characterised in that the second filter is composed of charged material to absorb more free compound.

8. A filter according to Claim 5, 6 or 7, characterised in that the second filter is composed or coated with DEAE, cellulose phosphate or carboxymethyl cellulose.

9. A filter according to Claim 8, characterised in that the second filter is coated with monoclonal antibodies or complementary sequence for nucleic acids.

10. A filter according to any one of Claims 5 to 9, characterized in that the first filter is composed of charged material to repel free compound and reduce non-specific binding.

11. A filter according to any one of Claims 5 to 10, characterised in that the first filter is composed of hydrophobic material to reduce non-specific binding of hydrophilic free compounds.

12. A filter according to any one of Claims 5 to 11, characterised in that the filters are coated with or composed of scintillants of different characteristics so that labelled compounds incorporated into particles borne on a first filter will give different light characteristics compared with the free compound bound by a subsequent filter.

## Patentansprüche

1. Verfahren zum Filtern in Tests mit konkurrierenden Radioisotopen, gekennzeichnet durch den Einsatz von mindestens zwei Filtern für die gleiche Probe, so daß die radioaktive Zählrate des ersten Filters die Aktivität der Teilchen anzeigt und jene eines nachfolgenden Filters eine Abschätzung von nicht spezifischer Bindung ergibt.

2. Verfahren nach Anspruch 1, gekennzeichnet durch die Benutzung des Verhältnisses der Radioaktivität der Filter als eine Abschätzung von gebundenem gegenüber freiem Material, die mit Standardproben verglichen werden soll.

3. Verfahren nach Anspruch 1 oder 2, dadurch gekennzeichnet, daß die Filter mit unterschiedlich zusammengesetzten Szintillationsmitteln beschichtet sind oder daraus bestehen, so daß markierte Verbindungen, die in Teilchen eingearbeitet sind, welche auf einem ersten Filter getragen werden, Lichteigenschaften ergeben, die von jenen der freien Verbindung, die durch einen nachfolgenden Filter gebunden ist, verschieden sind.

4. Verfahren nach einem der Ansprüche 1 bis 3, gekennzeichnet durch Trocknen und Erhitzen der Filter nach dem Filtern, jedoch vor dem Zählen, um die Zählwirksamkeit zu erhöhen.

5. Filter zum Filtern in Tests mit konkurrierenden Radioisotopen, dadurch gekennzeichnet, daß der Filter mindestens zwei Filter aufweist, die für die gleiche Probe benutzt werden, so daß die radioaktive Zählrate des ersten Filters die Aktivität der Teilchen anzeigt und jene eines nachfolgenden Filters eine Abschätzung von nichtspezifischer Bindung ergibt.

6. Filter nach Anspruch 5, dadurch gekennzeichnet, daß der zweite Filter dicker als der erste ist, damit mehr freie Verbindung absorbiert und die Zählgenauigkeit erhöht wird, wobei die Verbindungen organische, anorganische oder biologische Verbindungen, Elemente oder Ionen sind.

7. Filter nach Anspruch 5 oder 6, dadurch gekennzeichnet, daß der zweite Filter aus einem geladenen Material besteht, um mehr freie Verbindung zu vermindern.

8. Filter nach Anspruch 5, 6 oder 7, dadurch gekennzeichnet, daß der zweite Filter aus DEAE, Cellulosephosphat oder Carboxymethylcellulose besteht oder damit beschichtet ist.

9. Filter nach Anspruch 8, dadurch gekennzeichnet, daß der zweite Filter mit monoklonalen Antikörpern oder einer komplementären Sequenz für Nucleinsäuren beschichtet ist.

10. Filter nach einem der Ansprüche 5 bis 9, dadurch gekennzeichnet, daß der erste Filter aus einem geladenen Material besteht, um freie Verbindungen abzustoßen und nichtspezifische Bindungen zu vermindern.

11. Filter nach einem der Ansprüche 5 bis 10, dadurch gekennzeichnet, daß der erste Filter aus einem hydrophoben Material besteht, um nichtspezifische Bindungen hydrophiler freier Verbindungen zu vermindern.

12. Filter nach einem der Ansprüche 5 bis 11, dadurch gekennzeichnet, daß die Filter mit Szintillationsmitteln verschiedener Eigenschaften beschichtet sind oder daraus bestehen, so daß markierte Verbindungen, die in Teilchen eingearbeitet sind, welche auf einem ersten Filter getragen werden, Lichteigenschaften ergeben, die im Vergleich zu der freien Verbindung, die durch einen nachfolgenden Filter gebunden ist, verschieden sind.

## Revendications

1. Procédé de filtration dans un test de compétition avec isotopes radioactifs, caractérisé en ce que deux filtres au moins sont utilisés pour le même échantillon, de sorte que la radioactivité mesurée sur le premier filtre indiquera l'activité des particules, et que la radioactivité mesurée sur un filtre suivant fournira une estimation pour la liaison non-spécifique.

2. Procédé selon la revendication 1, caractérisé en ce que le rapport des radioactivités des filtres est utilisé comme estimation du rapport matière liée/matière libre par comparaison avec des échantillons de référence.

3. Procédé selon la revendication 1 ou la revendication 2, caractérisé en ce que les filtres sont recouverts, ou composés, de scintillateurs de compositions différentes, de sorte que les composés marqués incorporés dans des particules portées par un premier filtre donneront des caractéristiques lumineuses différentes de celles du composé libre lié à un filtre suivant.

4. Procédé selon l'une quelconque des revendications 1 à 3, caractérisé en ce que les filtres sont séchés et chauffés après la filtration mais avant le comptage, afin d'augmenter l'efficacité de comptage.

5. Filtre pour la filtration dans un test de compétition avec isotopes radioactifs, caractérisé en ce que le filtre comprend deux filtres au moins à utiliser pour le même échantillon, de sorte que la radioactivité mesurée sur le premier filtre indiquera l'activité des particules, et que la radioactivité mesurée sur un filtre suivant fournira une estimation pour la liaison non-spécifique.

6. Filtre selon la revendication 5, caractérisé en ce que le second filtre est plus épais que le premier, afin d'absorber une plus grande quantité de composé libre et d'améliorer la précision du comptage, que les composés soient des composés, des éléments ou des ions organiques, inorganiques ou biologiques.

7. Filtre selon la revendication 5 ou la revendication 6, caractérisé en ce que le second filtre est fait de matière chargée afin d'absorber une plus grande quantité de composé libre.

8. Filtre selon l'une quelconque des revendications 5 à 7, caractérisé en ce que le second filtre est fait, ou recouvert, de DEAE, de phosphate de cellulose ou de carboxyméthyle cellulose.

9. Filtre selon la revendication 8, caractérisé en ce que le second filtre est recouvert d'anticorps monoclonaux ou de séquences complémentaires d'acides nucléiques.

10. Filtre selon l'une quelconque des revendications 5 à 9, caractérisé en ce que le premier filtre est fait de matière chargée, afin de repousser les composés libres et de diminuer la liaison non-spécifique.

11. Filtre selon l'une quelconque des revendications 5 à 10, caractérisé en ce que le premier filtre est fait de matière hydrophobe, afin de diminuer la liaison non-spécifique des composés libres hydrophiles.

12. Filtre selon l'une quelconque des revendications 5 à 11, caractérisé en ce que les filtres sont recouverts, ou composés, de scintillateurs de caractéristiques différentes, de sorte que les composés marqués incorporés dans des particules portées par un premier filtre donneront des caractéristiques lumineuses différentes de celles du composé libre lié à un filtre suivant.
